# EUROPEAN PATENT APPLICATION

(11) **EP 4 523 707 A1**
(43) Date of publication of application: **19.03.2025**
(21) Application number: 23806871.2
(22) Date of filing: 15.05.2023
(51) Int. Cl.: A61K 48/00, C07K 16/28, C07K 16/30, C12N 15/09, A61P 35/00

(54) **GENE HAVING EXTRACELLULAR DOMAIN OF BCMA AS LABEL, POLYPEPTIDE, RECOMBINANT EXPRESSION VECTOR, GENETICALLY ENGINEERED CELL AND USE THEREOF**

(30) Priority: 20.05.2022 CN 202210550548
(71) Applicant: Shanghai Yake Biotechnology Ltd., Shanghai 200438 (CN)
(72) Inventor: CHANG, Alex Hongsheng, Shanghai 200438 (CN); ZHANG, Yanlei, Shanghai 200438 (CN)
(74) Representative: Bayramoglu et al.
(86) International application number: PCT/CN2023/094184
(87) International publication number: WO 2023/221919

(57) **Abstract**

The present disclosure provides a gene using a BCMA extracellular domain as a marker, a polypeptide, a recombinant expression vector, a genetically engineered cell, and use thereof. The gene tBCMA is a gene encoding a complete or partial sequence of the BCMA extracellular domain, or a gene encoding a sequence at least 85% identical to the complete or partial sequence. The present disclosure uses an extracellular domain polypeptide (tBCMA) of a B cell maturation antigen as a marker for the technology of detecting and removing the genetically engineered immune cells. The present disclosure can be widely used for the detection of various genetically engineered immune cells, thereby effectively solving the problems of detection of the genetically engineered immune cells after preparation and infusion, and providing a feasible strategy when these cells need to be removed due to serious toxic and side effects in clinical therapy.

## Description

### TECHNICAL FIELD

The present disclosure relates to the technical field of cellular immunotherapy, and in particular, to a gene tBCMA for BCMA extracellular domain, an encoded tBCMA polypeptide, a recombinant expression vector, a genetically engineered cell, and use thereof.

### BACKGROUND TECHNOLOGY

Genetically engineered immune cells, e.g., CAR-T cells, are very important for detection after preparation and infusion. The detection of the genetically engineered immune cells determines the correct dose for infusion, monitors the duration and amplification of infusion cells in vivo, and is closely related to the evaluation of therapeutic effects and the intervention of toxic and side effects. In addition, strategies to remove infused immune cells are also very important when it is necessary to remove these cells in time due to serious toxic and side effects in clinical therapy.

A marker gene can perform the efficient detection and positive screening of the genetically engineered immune cells. In particular, when some cell surface proteins that are not easy to detect are expressed on the genetically engineered immune cells, it causes difficulty in detection during preparation and tracing after infusion of these immune cells, and it is especially important to use the marker gene. In addition, when it is necessary to remove infused immune cells due to severe toxic and side effects in clinical therapy, for example, allogeneic T cell infusion causes graft-versus-host disease or the infusion of CAR-T cells result in severe toxic and side effects, a protein expressed from the marker gene can be used as a target for removing these infused immune cells.

Current commonly used methods for monitoring CAR-T cells are primarily directed to the detection of an extracellular antigen recognition domain of a CAR consisting of single-chain antibodies. The detection methods typically use protein L, Goat anti-human/mouse IgG F(ab')2 fragment, or Antigen-Fc. These detection methods often fail to fully meet the need for detection of the genetically engineered immune cells due to lack of specificity and sensitivity.

Due to the importance of the marker gene on the genetically engineered immune cells, at present, a variety of marker genes have been applied in scientific research and clinical trials, including a variety of truncated type I transmembrane proteins such as CD19, CD34 and EGFR. One feature of CD19 and CD34 is that the existing Miltenyi CliniMACS screening system is available for clinical grade sorting. A human epidermal growth factor receptor (EGFR) is a tyrosine kinase receptor of the growth factor receptor ErbB family, and is not expressed on cells of hematopoietic and lymphatic systems. Cetuximab (Erbitux) is an antibody drug targeting EGFR, and has been cleared by the U.S. FDA for the therapy of metastatic colorectal cancer and head and neck cancer. A truncated EGFR (EGFRt) can serve as a marker for the genetically engineered immune cells. In EGFRt, the extracellular domains I and II and intracellular signal domain thereof are deleted, and the cetuximab binding site of the extracellular domain III thereof is retained. EGFRt can be used for immunomagnetic enrichment and flow cytometry and immunohistochemical detection by genetically engineered T cells as markers, and removing in-vivo genetically engineered T cells with the cetuximab. Although these above cell surface proteins can serve as marker genes, they all have a relatively large molecular weight, thereby possibly increasing the load of a gene vector and reducing the transduction efficiency of the vector, and affecting the expression density of a gene modified protein on T cells, the target antigen binding ability and the tumor killing effect.

The detection of the genetically engineered immune cells is very important for both preparation of immune cells and observation after infusion, determines the correct dosage of infusion cells, monitors the duration and amplification of the infusion cells in vivo, and is closely related to the evaluation of therapeutic effects and the intervention of toxic and side effects. However, the existing detection methods cannot sufficiently meet the need for detection of the genetically engineered immune cells due to lack of specificity and sensitivity. However, because these marker genes have a relatively large molecular weight, the existing methods for detecting genetically engineered immune cells using marker genes possibly increase the load of a gene vector and reduce the transduction efficiency of the vector, and affect the target antigen binding ability of genes to the genetically engineered immune cells and the tumor killing effect. In addition, strategies to remove the infusion cells are also very important when it is necessary to remove these cells in time due to serious toxic and side effects in clinical therapy.

### CONTENT OF THE INVENTION

Aiming at the defects in the prior art, the purposes of the present disclosure are to provide a gene tBCMA for BCMA extracellular domain, an encoded tBCMA polypeptide, a recombinant expression vector, a genetically engineered cell, and use thereof. A tBCMA polypeptide is mainly used as a marker for detecting and removing genetically engineered immune cells.

The purposes of the present disclosure are implemented through the following technical solutions:

A first aspect of the present disclosure is to provide a gene tBCMA for BCMA extracellular domain, the gene tBCMA is a gene encoding a complete or partial sequence of BCMA extracellular domain, or a gene encoding a sequence at least 85% identical to the complete or partial sequence, a nucleotide sequence of a gene BCMA is as shown in SEQ ID NO. 1, and a complete nucleotide sequence of the gene tBCMA is as shown in SEQ ID NO. 2.

A second aspect of the present disclosure is to provide a tBCMA polypeptide for BCMA extracellular domain, the tBCMA polypeptide is a polypeptide encoded by the gene tBCMA for BCMA extracellular domain according to the first aspect, a polypeptide amino acid sequence encoded by BCMA is as shown in SEQ ID NO. 3, and a complete polypeptide amino acid sequence encoded by the gene tBCMA is as shown in SEQ ID NO. 4.

A third aspect of the present disclosure is to provide a recombinant expression vector, and the recombinant expression vector includes the nucleotide sequence of the gene tBCMA according to the first aspect.

A fourth aspect of the present disclosure is to provide a genetically engineered cell, and the genetically engineered cell includes the gene tBCMA for BCMA extracellular domain according to the first aspect.

In another preferred embodiment, the genetically engineered cell is an immune cell modified by genetic engineering.

In another preferred embodiment, the immune cell includes, but is not limited to, a T cell, a γδT cell, an NK cell, an NKT cell or a macrophage; and a T cell, a γδT cell, an NK cell, an NKT cell or a macrophage from induced pluripotent stem cells.

In another preferred embodiment, the T cell is a CAR-T cell, and the CAR-T cell includes the gene tBCMA for BCMA extracellular domain according to the first aspect as a marker gene.

The CAR-T cell is a CAR-T cell where an antigen binding domain targets any of the following targets including but not limited to: CD1a, CD2, CD4, CD5, CD7, CD19, CD20, CD22, CD30, CD32b, CD33, CD34, CD37, CD38, CD44v6, CD56, CD70, CD79b, CD83, CD117, CD123, CD133, CD138, CD155, CD171, CD276, CD319, CD371, Chlorotoxin, FLT3, Folate receptor beta, GPRC5D, IL1RAP, Lag-3, LewY, Sigelec-6, LILRB4, LMP1, B7H6, NKG2DLs, PD-L1, ROR1, TIM3, CEA, Claudin-6, Claudin-18.2, EGFR, EGFRvIII, EpCam, EphA2, Fibroblast activation protein alpha, GTPase-activating protein, GD2, Glypican-1, GPC2, GPC3, Her2, IL13Ra2, Mesothelin, PSCA, PSMA, Muc1, and TAG-72.

In another preferred embodiment, the CAR-T cell is a CAR-T cell where CD27 as an antigen binding domain targets CD70. An amino acid sequence of the CD70 is as shown in SEQ ID NO. 5.

In another preferred embodiment, the antigen binding domain is a complete or partial sequence of the CD27, or a sequence 85% identical to the complete or partial sequence of the antigen binding domain CD27. A complete amino acid sequence of the CD27 is as shown in SEQ ID NO. 6.

In another preferred embodiment, the CAR-T cell is a CAR-T cell where a CD30 ligand as an antigen binding domain targets CD30. An amino acid sequence of the CD30 is as shown in SEQ ID NO. 7.

In another preferred embodiment, the antigen binding domain is a complete or partial sequence of the CD30 ligand, or a sequence 85% identical to the complete or partial sequence of the antigen binding domain CD30 ligand. A complete amino acid sequence of the CD30 ligand (CD30LR) is as shown in SEQ ID NO. 8.

In another preferred embodiment, the CAR-T cell is a CAR-T cell where a CD276 ligand (CD276L) as an antigen binding domain targets CD276. An amino acid sequence of the CD276 is as shown in SEQ ID NO. 9.

In another preferred embodiment, the antigen binding domain is a complete or partial sequence of the CD276 ligand, or a sequence 85% identical to the complete or partial sequence of the antigen binding domain CD276 ligand. The CD276L is derived from a cell binding ligand targeting the CD276 (Stern LA, Lown PS, Kobe AC, Abou-Elkacem L, Willmann JK, Hackel BJ. Cellular-Based Selections Aid Yeast-Display Discovery of Genuine Cell-Binding Ligands: Targeting Oncology Vascular Biomarker CD276. ACS Comb Sci. 2019 Mar 11;21(3):207-222.). A complete amino acid sequence of the CD276 ligand (CD276L) is as shown in SEQ ID NO. 10.

In another preferred embodiment, the CAR-T cell is a CAR-T cell where IL3 as an antigen binding domain targets CD123. An amino acid sequence of the CD123 is as shown in SEQ ID NO. 11.

In another preferred embodiment, the antigen binding domain is a complete or partial sequence of the IL3, or a sequence 85% identical to the complete or partial sequence of the antigen binding domain IL3. A complete amino acid sequence of the IL3 is as shown in SEQ ID NO. 12.

In another preferred embodiment, the CAR-T cell is a CAR-T cell where a CD20 single-chain antibody as an antigen binding domain targets CD20. An amino acid sequence of the CD20 is as shown in SEQ ID NO. 13.

In another preferred embodiment, the antigen binding domain is a complete or partial sequence of the CD20 single-chain antibody, or a sequence 85% identical to the complete or partial sequence of the antigen binding domain CD20 single-chain antibody, the CD20 single-chain antibody is derived from a monoclonal antibody rituximab (Maloney DG, Grillo-López AJ, White CA, Bodkin D, Schilder RJ, Neidhart JA, Janakiraman N, Foon KA, Liles TM, Dallaire BK, Wey K, Royston I, Davis T, Levy R. IDEC-C2B8 (Rituximab) anti-CD20 monoclonal antibody therapy in patients with relapsed low-grade non-Hodgkin's lymphoma. Blood. 1997 Sep 15;90(6):2188-95.), and a complete amino acid sequence of the CD20 single-chain antibody (CD20 scFv) is as shown in SEQ ID NO. 14.

In another preferred embodiment, in the CAR-T cell, a tBCMA polypeptide expressed from the gene tBCMA for BCMA extracellular domain is capable of being directly linked to an antigen binding domain of a CAR to form a fusion protein.

In another preferred embodiment, in the CAR-T cell, the tBCMA polypeptide expressed from the gene tBCMA for BCMA extracellular domain is linked to an antigen recognition region of the CAR via a linker peptide to form a fusion protein. An amino acid sequence of the linker peptide includes but is not limited to: e.g., SEQ ID NO. 15, SEQ ID NO. 16, SEQ ID NO. 17 or SEQ ID NO. 18.

In another preferred embodiment, in the CAR-T cell, the tBCMA polypeptide expressed from the gene tBCMA for BCMA extracellular domain is linked via a 2A peptide (ribosomal codon skip site), and expressed simultaneously with the CAR on the CAR-T cell membrane. The ribosomal codon skip site short chain polypeptide may be any 2A peptide sequence, including but not limited to the following sequences: Thosea asigna virus, T2A, Foot-and-mouth disease virus, F2A, and porcine teschovirus-1, P2A. Amino acid sequences of the above 2A peptides are as shown in SEQ ID NO. 19, SEQ ID NO. 20 or SEQ ID NO. 21, respectively.

In another preferred embodiment, in the CAR-T cell, the gene tBCMA for BCMA extracellular domain is linked via an IRES (internal ribosome entry site), and expressed simultaneously with the CAR on the CAR-T cell membrane.

In another preferred embodiment, in the CAR-T cell, the tBCMA polypeptide expressed from the gene tBCMA for BCMA extracellular domain is expressed, via a gene editing method, simultaneously with the CAR on the CAR-T cell membrane. Gene editing tools used by the gene editing method include but are not limited to transcription activator-like effector nucleases (TALENs) or clustered regularly interspaced short palindromic repeats/CRISPR-associated protein 9 (CRISPR/Cas9).

In another preferred embodiment, in the CAR-T cell, the tBCMA polypeptide expressed from the gene tBCMA for BCMA extracellular domain is expressed, via multi-vector co-transduction or sequential transduction, simultaneously with the CAR on the CAR-T cell membrane.

In another preferred embodiment, the NK cell is a CAR-NK cell, and the CAR-NK cell includes the gene tBCMA for BCMA extracellular domain according to the first aspect as a marker gene.

A fifth aspect of the present disclosure is to provide use of the gene tBCMA for BCMA extracellular domain according to the first aspect in detecting a genetically engineered cell, and the genetically engineered cell includes the gene tBCMA for BCMA extracellular domain according to the first aspect.

In another preferred embodiment, detection of the genetically engineered cell is completed by a detection reagent specifically recognizing a tBCMA marker gene.

In another preferred embodiment, the detection reagent includes a small molecular compound, a polypeptide, a protein, or an antibody.

In another preferred embodiment, the genetically engineered cell is an immune cell modified by genetic engineering.

In another preferred embodiment, the immune cell includes, but is not limited to, a T cell, a γδT cell, an NK cell, an NKT cell or a macrophage; and a T cell, a γδT cell, an NK cell, an NKT cell or a macrophage from induced pluripotent stem cells.

In another preferred embodiment, the T cell is a CAR-T cell, and the CAR-T cell includes the gene tBCMA for BCMA extracellular domain according to the first aspect as a marker gene.

In another preferred embodiment, the NK cell is a CAR-NK cell, and the CAR-NK cell includes the gene tBCMA for BCMA extracellular domain according to the first aspect as a marker gene.

A sixth aspect of the present disclosure is to provide use of the gene tBCMA for BCMA extracellular domain according to the first aspect in removing a genetically engineered cell, and the genetically engineered cell includes the gene tBCMA for BCMA extracellular domain according to the first aspect.

In another preferred embodiment, removing of the genetically engineered cell is completed by a removing reagent specifically recognizing a tBCMA marker gene.

In another preferred embodiment, the removing reagent includes a small molecular compound, a polypeptide, a protein, an antibody, or a genetically engineered immune cell targeting tBCMA.

In another preferred embodiment, the genetically engineered cell is an immune cell modified by genetic engineering.

In another preferred embodiment, the immune cell includes, but is not limited to, a T cell, a γδT cell, an NK cell, an NKT cell or a macrophage; and a T cell, a γδT cell, an NK cell, an NKT cell or a macrophage from induced pluripotent stem cells.

In another preferred embodiment, the T cell is a CAR-T cell, and the CAR-T cell includes the gene tBCMA for BCMA extracellular domain according to the first aspect as a marker gene.

In another preferred embodiment, the NK cell is a CAR-NK cell, and the CAR-NK cell includes the gene tBCMA for BCMA extracellular domain according to the first aspect as a marker gene.

The present disclosure will use a tBCMA polypeptide as a marker for detecting and removing genetically engineered immune cells. The genetically engineered cells may be any immune cell modified by genetic engineering, including but not limited to a T cell, a γδT cell, an NK cell, an NKT cell or a macrophage; and a T cell, a γδT cell, an NK cell, an NKT cell or a macrophage from induced pluripotent stem cells. The tBCMA polypeptide may be a complete or partial sequence of BCMA extracellular domain, and may be linked, directly or via any length of a linker peptide, to an antigen recognition domain of a CAR for marking. The tBCMA polypeptide may also carry a transmembrane domain of itself or from other membrane proteins, and is expressed, by means of a 2A peptide, an IRES, gene editing, and multi-vector co-transduction or sequential transduction, simultaneously with the CAR on a cell membrane, thereby marking immune cells modified by genetic engineering.

Compared with the prior art, the present disclosure has the following beneficial effects:

1) Compared with a large molecular marker gene, an extracellular domain using BCMA only has 54 aa, which is several times smaller than CD19 and EGFR used as a marker gene, thus, the gene load can be effectively reduced when a gene vector is constructed, so that the gene vector has a greater advantage in the packaging capacity, transduction efficiency and the killing capacity of CAR-T cells.

2) A tBCMA is derived from the extracellular domain of BCMA, and since BCMA is a protein expressed from the human body itself, it has no immunogenicity for the human. Thus, upon infusion of genetically engineered cells carrying the tBCMA as a marker, an immune response to a marker protein will not be caused.

3) BCMA is expressed mainly when B cells are further differentiated into plasmocytes, and rarely expressed on hematopoietic stem cells, unmodified T cells and immature B cells. Thus, the tBCMA, as a marker, can be used for the removing of the genetically engineered cells after infusion, without causing damage to vital tissues and organs of the human body.

Therefore, the present disclosure uses the tBCMA as the marker gene to provide a better method for detecting and removing genetically engineered immune cells, so that a vector carrying the marker gene can be further enhanced in terms of packaging capacity and transduction efficiency, the current problem of removing the genetically engineered cells after detection and infusion can be solved, and the genetically engineered immune cells have a greater advantage in terms of tumor killing capacity. Currently, a variety of drugs targeting BCMA, including antibody-drug conjugate (ADC), bispecific T cell engager (BiTE) and chimeric antigen receptor modified T cell (CAR-T) therapy, are all in active clinical development. Recently, the U.S. FDA approved BCMA-targeting ADC drug, i.e., Belantamab mafodotin-blmf (GSK2857916), and BCMA-targeting CAR-T cell therapy, i.e., Abecma (idecabtagene vicleucel), for the therapy of relapsed or refractory multiple myeloma. These drugs can be used effectively for removing genetically engineered cells using the tBCMA as a marker during infusion.

### DESCRIPTION OF THE DRAWINGS

By reading the detailed description of non-limiting embodiments with reference to the following drawings, the other features, objectives, and advantages of the present disclosure will become more apparent:

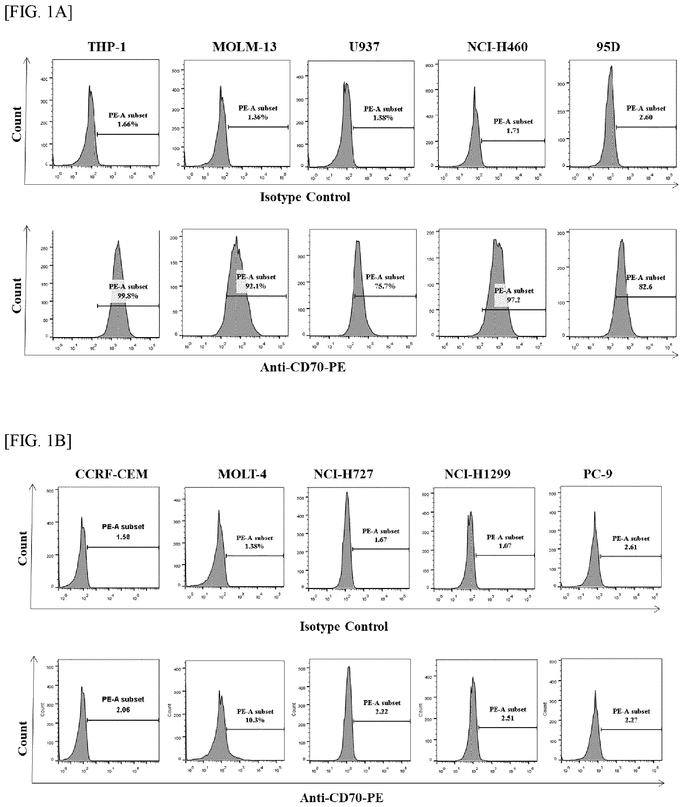

FIG. 1A shows expression results of flow cytometry detection of CD70 on tumor cell lines as follows: myeloid leukemia cell lines THP-1, MOLM-13 and U937, and hepatoma cell lines NCI-H460 and 95D, where the CD70 is expressed as positive on the above tumor cell lines; and a CD70 flow cytometry detection reagent is Anti-CD70-PE.
FIG. 1B shows expression results of flow cytometry detection of CD70 on tumor cell lines as follows: T-cell leukemia cell lines CCRF-CEM, MOLT-4, and hepatoma cell lines NCI-H727, NCI-H1299 and PC-9, where the CD70 is expressed as negative on the above tumor cell lines; and a CD70 flow cytometry detection reagent is Anti-CD70-PE.

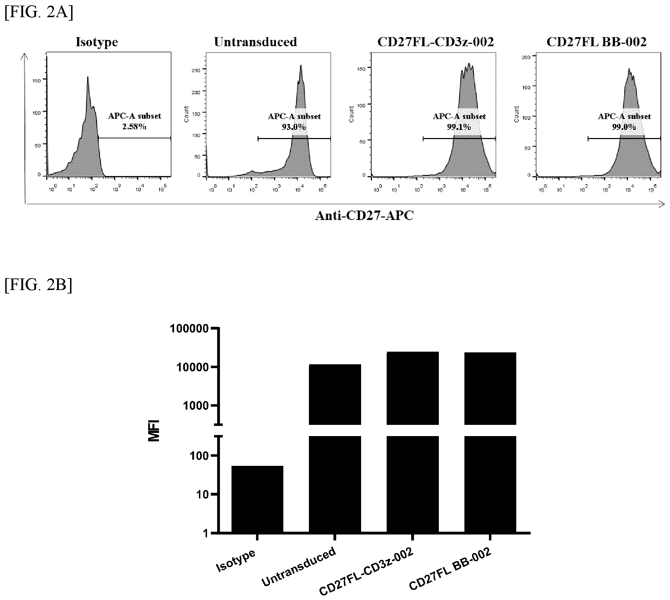

FIG. 2A shows expression results of flow cytometry detection of CD27 on untransduced T cells and CD27FL-CD3z-002 and CD27FL BB-002 CAR-T cells using CD27 as an antigen binding domain, where a CD27 flow cytometry detection reagent is Anti-CD27-APC.
FIG. 2B is a histogram of MFI of the flow cytometry detection of the CD27 in FIG. 2A, where results show that the CD27 is also highly expressed on the untransduced T cells.

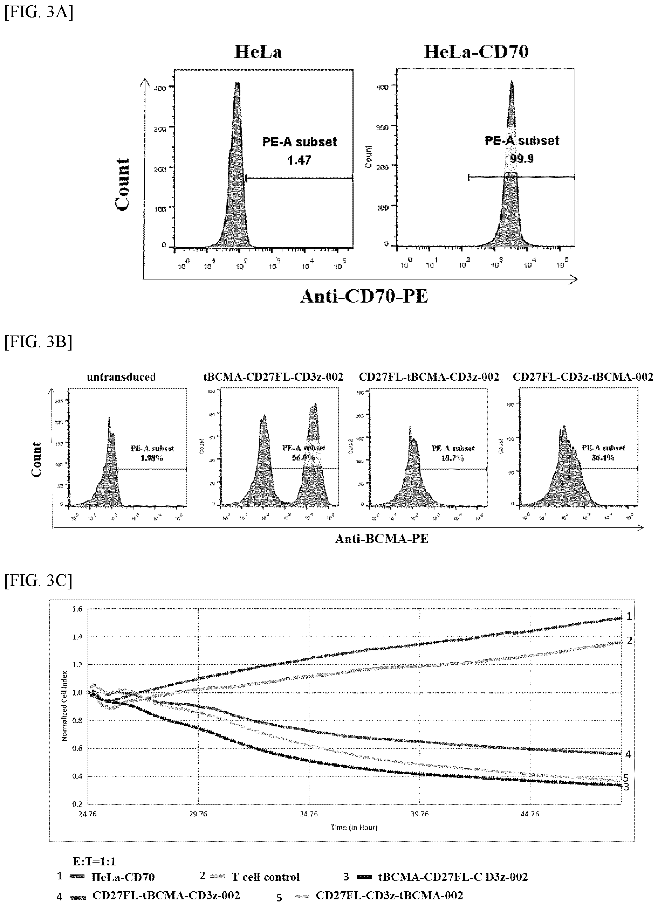

FIG. 3A shows a genetically modified HeLa cell line and a HeLa-CD70 cell line expressing CD70 obtained by flow cytometry sorting, where a CD70 flow cytometry detection reagent is Anti-CD70-PE.
FIG. 3B shows expression results of flow cytometry detection of tBCMA-CD27FL-CD3z-002, CD27FL-tBCMA-CD3z-002 and CD27FL-CD3z-tBCMA-002 using a tBCMA polypeptide as a marker on T cells, where a flow cytometry detection reagent for tBCMA polypeptide-marked CAR-T cells is Anti-BCMA-PE.
FIG. 3C is a curve graph of results of in-vitro killing assays performed by an iCELLigence^{™} real-time cell analyzer (Agilent Biosciences, Inc.), where HeLa-CD70 is a tumor cell line expressing CD70, T cell control is untransduced T cell control, and tBCMA-CD27FL-CD3z-002, CD27FL-tBCMA-CD3z-002 and CD27FL-CD3z-tBCMA-002 are CAR-T cells expressing tBCMA-CD27FL-CD3z-002, CD27FL-tBCMA-CD3z-002 and CD27FL-CD3z-tBCMA-002, respectively; and a target cell used for in-vitro cell killing assays is HeLa-CD70.

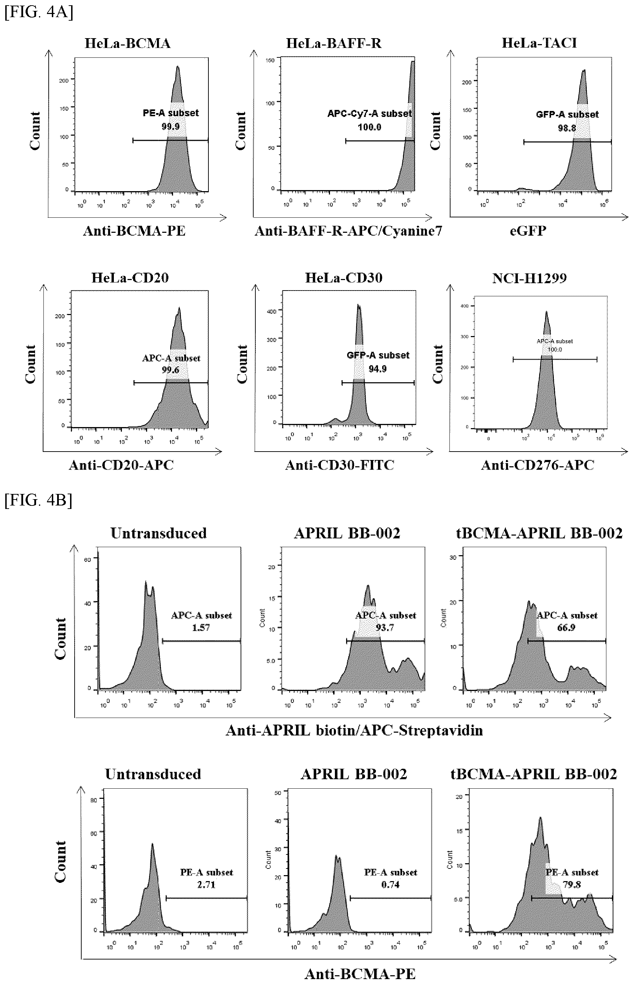

FIG. 4A is a diagram of flow cytometry detection of established HeLa cell lines expressing BCMA, BAFF-R, TACI, CD20 and CD30 and an NCI-H1299 cell line expressing CD276, where HeLa-BCMA, HeLa-TACI, HeLa-BAFF-R, HeLa-CD20, HeLa-CD30 and NCI-H1299 are tumor cell lines expressing BCMA, TACI, BAFF-R, CD20, CD30 and CD276, respectively; and flow cytometry detection reagents for tumor antigens BCMA, BAFF-R, TACI, CD20, CD30 and CD276 are Anti-human BCMA-PE (Biolegend, Cat#357503), anti-human BAFF-R-APC/Cyanine7 (Biolegend, Cat#316912), eGFP (TACI-expressed reporter gene), anti-human CD20-APC (BD, Cat#559776), anti-human CD30-FITC (BD, Cat#555829), and anti-human CD276-APC (Biolegend, Cat#351006), respectively.
FIG. 4B is a diagram of flow cytometry detection of a tBCMA-APRIL-BB-002 CAR-T cell using a tBCMA polypeptide as a marker, where an unmarked APRIL-BB-002 CAR-T cell is used as a control, and Untransduced represents untransduced T cells.

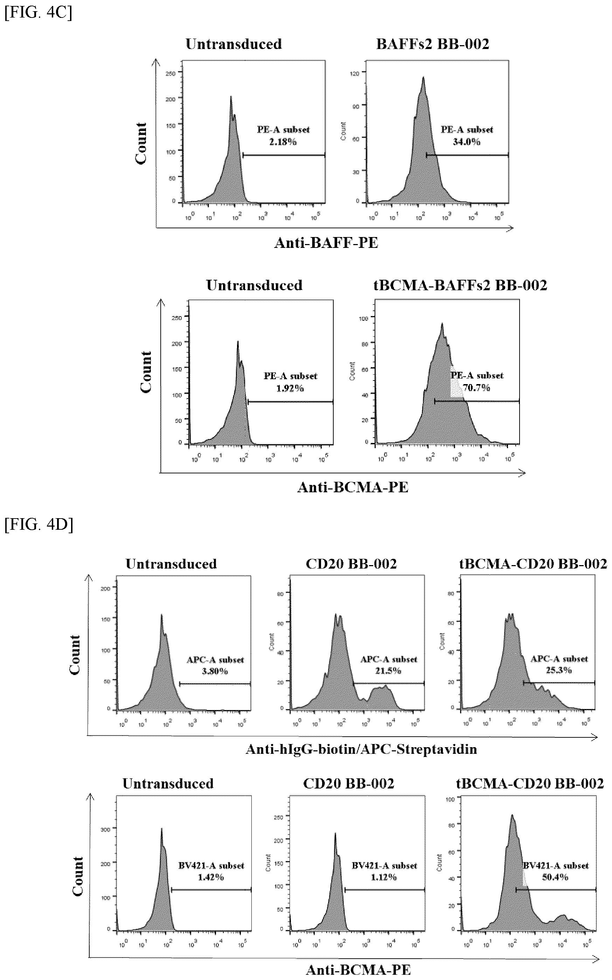

FIG. 4C is a diagram of flow cytometry detection of a tBCMA-BAFFs2-BB-002 CAR-T cell using a tBCMA polypeptide as a marker, where an unmarked BAFFs2-BB-002 CAR-T cell is used as a control, and Untransduced represents untransduced T cells.
FIG. 4D is a diagram of flow cytometry detection of a tBCMA-CD20 BB-002 CAR-T cell using a tBCMA polypeptide as a marker, where an unmarked CD20 BB-002 CAR-T cell is used as a control, and Untransduced represents untransduced T cells.

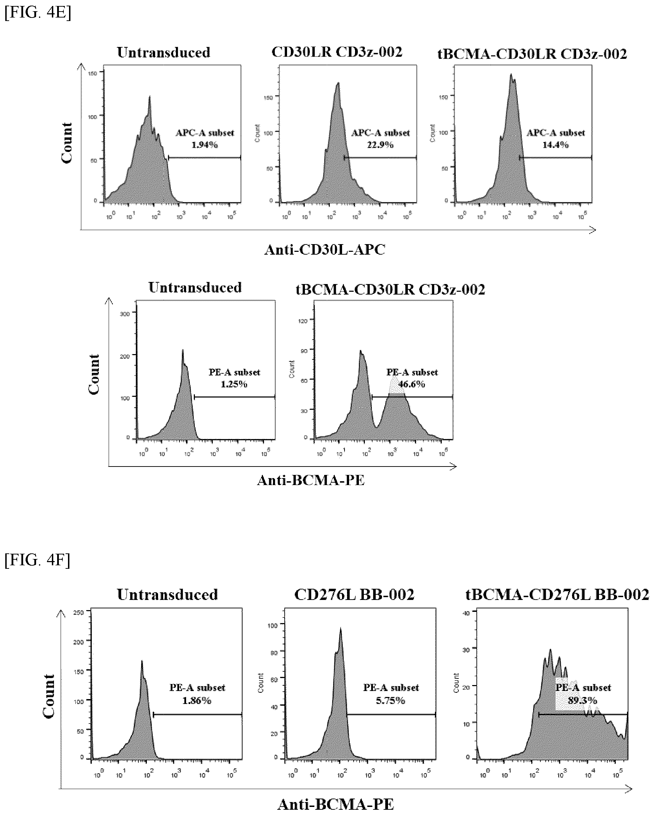

FIG. 4E is a diagram of flow cytometry detection of a tBCMA-CD30LR-CD3z-002 CAR-T cell using a tBCMA polypeptide as a marker, where an unmarked CD30LR-CD3z-002 CAR-T cell is used as a control, and Untransduced represents untransduced T cells.
FIG. 4F is a diagram of flow cytometry detection of a tBCMA-CD276L-BB-002 CAR-T cell using a tBCMA polypeptide as a marker, where an unmarked CD276L-BB-002 CAR-T cell is used as a control, and Untransduced represents untransduced T cells.

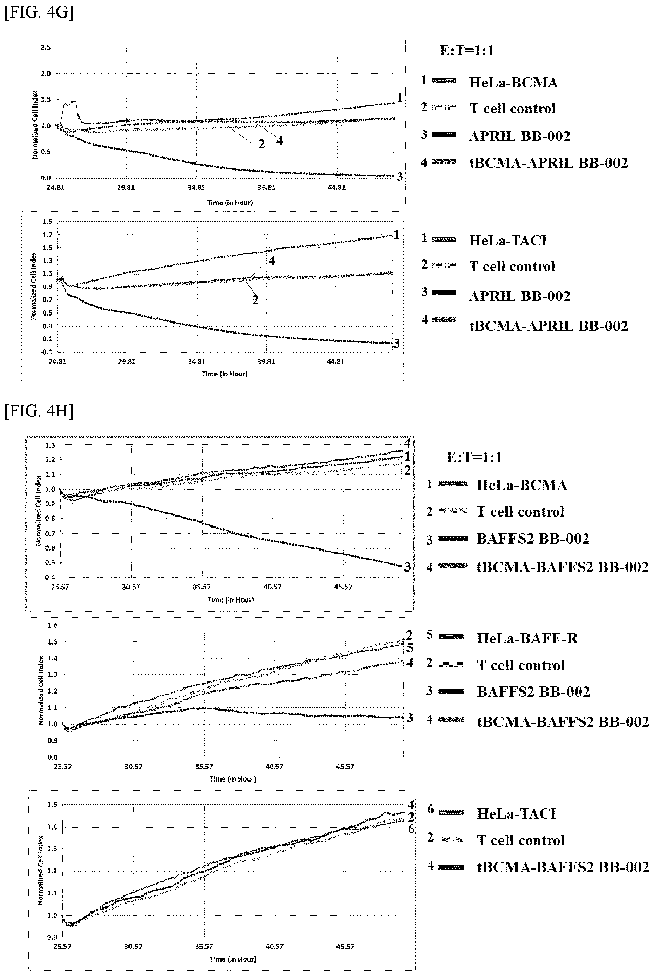

FIG. 4G is a curve graph of results of in-vitro killing assays of a tBCMA polypeptide-marked tBCMA-APRIL-BB-002 CAR-T cell detected by an iCELLigence^{™} real-time cell analyzer (Agilent Biosciences, Inc.), where HeLa-BCMA and HeLa-TACI are tumor cell lines expressing BCMA and TACI, respectively, T cell control is untransduced T cell control, tBCMA-APRIL-BB-002 is a tBCMA polypeptide-marked CAR-T cell, APRIL-BB-002 is an unmarked CAR-T cell, and target cells used are HeLa-BCMA and HeLa-TACI, respectively.
FIG. 4H is a curve graph of results of in-vitro killing assays of a tBCMA polypeptide-marked tBCMA-BAFFs2-BB-002 CAR-T cell detected by an iCELLigence^{™} real-time cell analyzer (Agilent Biosciences, Inc.), where HeLa-BCMA, HeLa-BAFF-R and HeLa-TACI are tumor cell lines expressing BCMA, BAFF-R and TACI, respectively, T cell control is untransduced T cell control, tBCMA-BAFFs2-BB-002 is a tBCMA polypeptide-marked CAR-T cell, and BAFFs2-BB-002 is an unmarked CAR-T cell; and target cells used are HeLa-BCMA, HeLa-TACI and HeLa-BAFF-R, respectively.

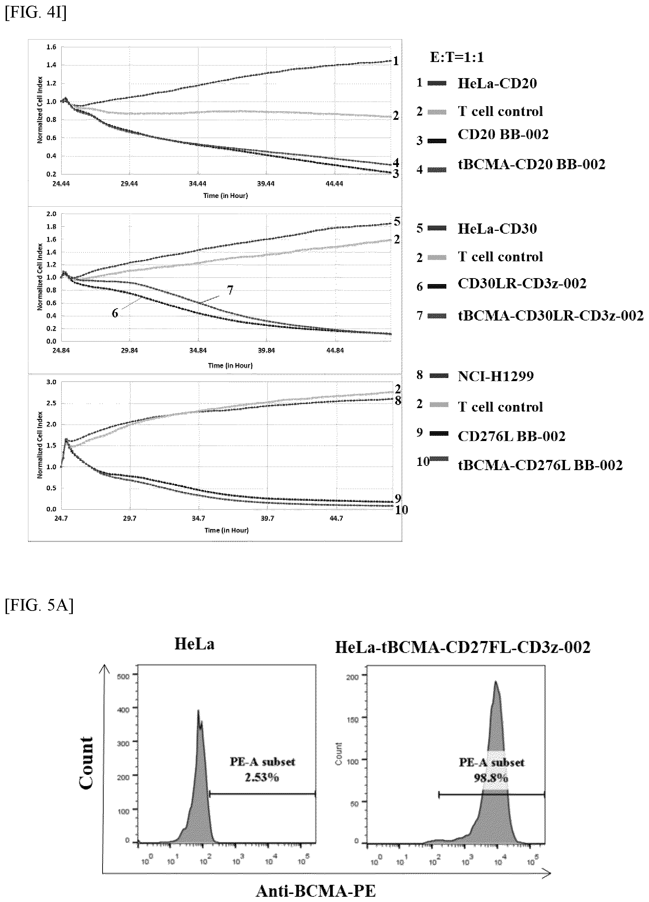

FIG. 4I is a curve graph of results of in-vitro killing assays of tBCMA polypeptide-marked tBCMA-CD20 BB-002, tBCMA-CD30LR-CD3z-002, tBCMA-CD276L-BB-002 CAR-T cells detected by an iCELLigence^{™} real-time cell analyzer (Agilent Biosciences, Inc.), where HeLa-CD20, HeLa-CD30 and NCI-H1299 are tumor cell lines expressing CD20, CD30 and CD276, respectively, T cell control is untransduced T cell control, and CD20 BB-002, CD30LR-CD3z-002 and CD276L-BB-002 are unmarked CAR-T cells; and target cells used are HeLa-CD20, HeLa-CD30 and NCI-H1299, respectively.
FIG. 5A shows an established CAR-modified tumor cell expressing a tBCMA polypeptide marker and targeting CD70, where a diagram of flow cytometry detection of HeLa-tBCMA-CD27FL-CD3z-002 is obtained by flow cytometry sorting, and a flow cytometry detection reagent is Anti-Human BCMA-PE (Biolegend, Cat#357503).

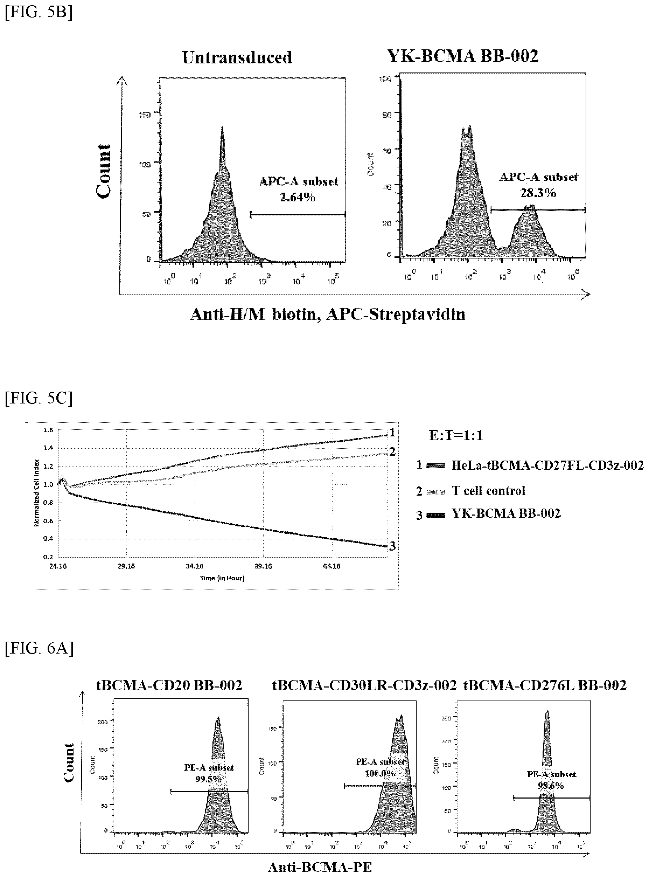

FIG. 5B is a diagram of flow cytometry detection of a prepared YK-BCMA BB-002 CAR-T cell using BCMA as a target and obtained by flow cytometry sorting, where a flow cytometry detection reagent is Anti-H/M biotin [the full name is Biotin-SP-conjugated AffiniPure F(ab')2 Fragment Goat Anti-Human IgG, Cat# 109-066-006, Biotin-SP-conjugated AffiniPure F(ab')2 Fragment Goat Anti-Mouse IgG, Cat#115-066-006, Jackson ImmunoResearch]; APC-Streptavidin, Cat#554067, BD.
FIG. 5C is a curve graph of results of in-vitro killing assays of a YK-BCMA BB-002 CAR-T cell on a HeLa-tBCMA-CD27FL-CD3z-002 tumor cell detected by an iCELLigence^{™} real-time cell analyzer (Agilent Biosciences, Inc.), where HeLa-tBCMA-CD27FL-CD3z-002 is a HeLa cell expressing a tBCMA polypeptide marker, i.e., a target cell used for in-vitro cell killing assays, YK-BCMA BB-002 is a CAR-T cell using BCMA as a target, and T cell control is untransduced T cell control. FIG. 6A is a diagram of flow cytometry detection of established CAR tumor cells HeLa-tBCMA-CD20-BB-002, HeLa-tBCMA-CD30LR-CD3z-002 and HeLa-tBCMA-CD276L-BB-002 expressing a tBCMA polypeptide marker and obtained by flow cytometry sorting, where a flow cytometry detection reagent is Anti-Human BCMA-PE (Biolegend, Cat#357503).

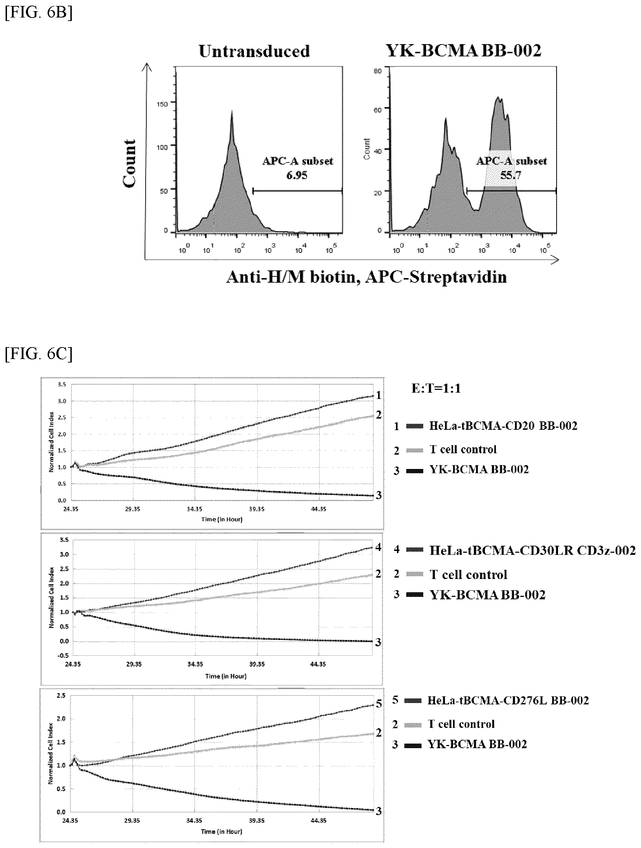

FIG. 6B is a diagram of flow cytometry detection of a prepared YK-BCMA BB-002 CAR-T cell using BCMA as a target and obtained by flow cytometry sorting, where a flow cytometry detection reagent is Anti-H/M biotin [the full name is Biotin-SP-conjugated AffiniPure F(ab')2 Fragment Goat Anti-Human IgG, Cat# 109-066-006, Biotin-SP-conjugated AffiniPure F(ab')2 Fragment Goat Anti-Mouse IgG, Cat#115-066-006, Jackson ImmunoResearch]; APC-Streptavidin, Cat#554067, BD.
FIG. 6C is a curve graph of results of in-vitro killing assays of a YK-BCMA BB-002 CAR-T cell on HeLa-tBCMA-CD20-BB-002, HeLa-tBCMA-CD30LR-CD3z-002 and HeLa-tBCMA- CD276L-BB-002 CAR tumor cells detected by an iCELLigence^{™} real-time cell analyzer (Agilent Biosciences, Inc.), where HeLa-tBCMA-CD20-BB-002, HeLa-tBCMA-CD30LR-CD3z-002 and HeLa-tBCMA- CD276L-BB-002 are CAR tumor cells using a tBCMA polypeptide as a marker, i.e., a target cell used for in-vitro cell killing assays, YK-BCMA BB-002 is a CAR-T cell using BCMA as a target, and T cell control is untransduced T cell control.

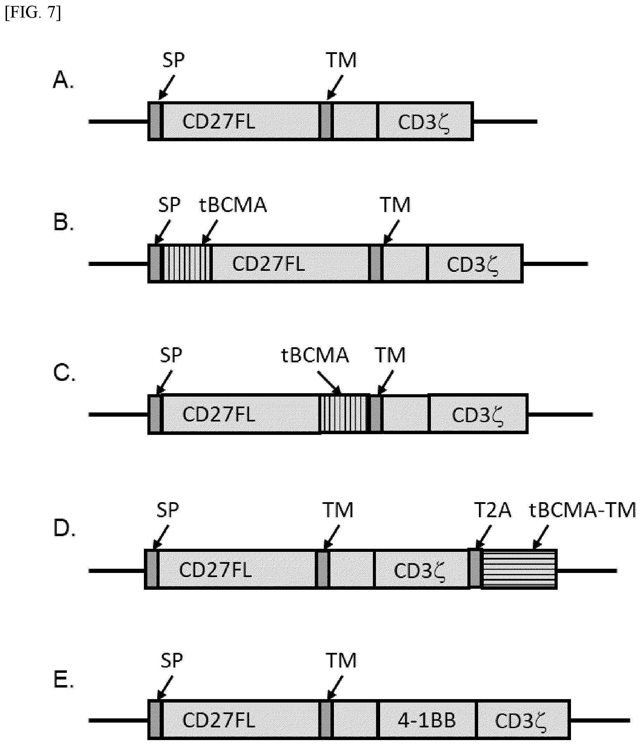

FIG. 7 is a schematic structural diagram of a CAR vector using a tBCMA polypeptide as a marker and targeting CD70, where A is a vector of CD27FL-CD3z-002 constructed by linking a ligand CD27 of CD70 to a CD3ζ T cell stimulation domain, where full-length CD27 (CD27FL) includes a CD27 signal peptide (SP), an extracellular domain, a transmembrane domain (TM), and an intracellular co-stimulation signal domain; B to D are three vectors using a tBCMA polypeptide as a marker and targeting CD70, where B. a tBCMA polypeptide of a tBCMA-CD27FL-CD3z-002 vector is located between the CD27 signal peptide (SP) and the CD27 extracellular domain; C. a tBCMA polypeptide of a CD27FL-tBCMA-CD3z-002 vector is located between the CD27 extracellular domain and the CD27 transmembrane domain; and D. a tBCMA polypeptide (tBCMA-TM) of a CD27FL-CD3z-tBCMA-002 vector carries its own transmembrane domain, and is linked, via T2A, to a CAR targeting CD70; and E. for a CD27FL BB-002 vector, a 4-1BB co-stimulation domain is added between CD27FL and a CD3ζ T cell stimulation domain.

### SPECIFIC IMPLEMENTATIONS

The present disclosure is illustrated in detail below in combination with specific embodiments. The following embodiments will assist those skilled in the art in further understanding the present disclosure, but do not limit it in any form. It shall be pointed out that for those of ordinary skill in the art, without departing from the concept of the present disclosure, several changes and improvements can also be made. These are within the protection scope of the present invention.

The present disclosure relates to a new technology for universal detection and removing of genetically engineered immune cells. The present disclosure uses a B cell maturation antigen (BCMA) extracellular domain polypeptide (tBCMA) as a marker for the technology of detecting and removing the genetically engineered immune cells. The present disclosure can be widely used for the detection of various genetically engineered immune cells, thereby effectively solving the problems of detection of the genetically engineered immune cells after preparation and infusion, and providing a feasible strategy when these cells need to be removed due to serious toxic and side effects in clinical therapy.

The present disclosure uses a tBCMA polypeptide as a marker for the detection and removing of genetically engineered cells. The genetically engineered cells may be any immune cell modified by genetic engineering, including but not limited to a T cell, a γδT cell, an NK cell, an NKT cell or a macrophage; and a T cell, a γδT cell, an NK cell, an NKT cell or a macrophage from induced pluripotent stem cells. The tBCMA polypeptide may be a complete or partial sequence of BCMA extracellular domain, or an amino acid sequence at least 85% identical to the complete or partial sequence of BCMA extracellular domain. The tBCMA polypeptide may be linked, directly or via any length of a linker peptide, to an antigen recognition domain of a CAR for marking. The tBCMA polypeptide may carry a transmembrane domain from itself or other membrane proteins. The tBCMA polypeptide may be expressed, by means of a 2A peptide, an internal ribosome entry site, gene editing, and multi-vector co-transduction, sequential transduction or other means, simultaneously with the CAR on a cell membrane, thereby marking immune cells modified by genetic engineering.

A B cell maturation antigen (BCMA), also known as TNFRSF17 or CD269, is one of the members of the Tumor necrosis factor receptor (TNFR) superfamily. BCMA has 184 amino acids (aa) in total, and is a 20.2-kDa type III membrane protein, including a 54 aa extracellular domain, a 23 aa transmembrane domain and a 107 aa intracellular domain. BCMA has two agonist ligands, including BAFF and a proliferation inducing ligand (APRIL), mainly secreted from bone marrow stromal cells, osteoclasts and macrophages in bone marrow in a paracrine manner. The APRIL binds with higher affinity to BCMA, compared with the BAFF. The APRIL also binds to TACI, while the BAFF binds more selectively restrictively to BAFF-R.

BCMA is expressed mainly when B cells are further differentiated into plasmocytes, and rarely expressed in hematopoietic stem cells or non-hematopoietic tissues, and is crucial for the survival of long-term-survival bone marrow plasmocytes (PCs), but does not affect the whole B cell homeostasis. The membrane-bound BCMA can be shed from a cell surface by γ secretase mediation, resulting in circulation of a soluble BCMA (sBCMA), and reducing the activation of the cell surface BCMA by the APRIL and the BAFF. BCMA may be recognized on the surfaces of almost all multiple myeloma (MM) cell lines (80%-100%), and is expressed more in malignant tumor plasmocytes than normal plasmocytes. Because of the restrictive high expression of BCMA in plasmocytes of normal humans and multiple myeloma patients, BCMA becomes an ideal target antigen for treating multiple myeloma.

BCMA represents a specific biomarker for normal and malignant plasmocytes. Currently, a variety of drugs targeting BCMA, including antibody-drug conjugate (ADC), bispecific T cell engager (BiTE) and chimeric antigen receptor modified T cell (CAR-T) therapy, are all in active clinical development. The ADC is a monoclonal antibody targeting a tumor-associated antigen (TAA), and is coupled, via a linker, to a toxic load, such as tubulin polymerization inhibitors, i.e., monomethyl auristatin F (MMAF) and pyrrolobenzodiazepine (PBD), or an RNA polymerase II inhibitor, i.e., α-amanitin. Upon binding to a target cell expressing the TAA, the ADC is internalized and releases a toxic load, to induce DNA damage and cell death of the target cell. The antibody-drug conjugate, anti-BCMA ADC GSK2857916, consists of a fucosylated and humanized IgG1 anti-BCMA monoclonal antibody coupled to the tubulin polymerization inhibitor MMAF. A BiTE^{®} molecule is a fusion protein consisting of a single-chain fragment variable (scFv) having unique antigen specificity. AMG 420, formerly known as BI 836909, is BCMA × CD3 BiTE^{®} molecule, and has been studied in patients with relapsed or refractory multiple myeloma. A variety of CAR-T cell therapies targeting BCMA have also shown efficacy in early clinical trials. Recently, the U.S. FDA approved BCMA-targeting ADC drug, i.e., Belantamab mafodotin-blmf (GSK2857916), and BCMA-targeting CAR-T cell therapy, i.e., Abecma (idecabtagene vicleucel), for the therapy of relapsed or refractory multiple myeloma.

The detection of genetically modified T cells, e.g., CAR-T cells, after both preparation and infusion is very important, determines the correct dose for infusion, monitors the duration and amplification of infusion cells, and is closely related to the therapeutic effects and the intervention of toxic and side effects. In addition, it is necessary to remove infused T cells in time due to serious toxic and side effects in clinical therapy, so selecting strategies to remove these cells is also very important. However, the currently commonly used detection methods, such as protein L, Jackson Ab, and Antigen-Fc following by secondary antibodies, cannot meet the need for the detection of all genetically modified T cells.

A marker gene can detect and positively screen genetically modified T cells. In particular, when some cell surface proteins that are not easy to detect are expressed on the genetically modified T cells, it causes difficulty in measurement of transduction efficiency and tracing after infusion of these T cells. In the above cases, the marker gene is very essential. In addition, it is necessary to remove infused T cells in time due to severe toxic and side effects in clinical therapy, for example, graft-versus-host disease or the infusion of CAR-T cells result in severe toxic and side effects, a marker protein on the surfaces of the infused T cells can be used as a target for removing these cells.

Marker genes that have been studied in scientific research and clinical trials include several truncated type I transmembrane proteins that are not normally expressed on T cells, including CD19, CD34 and EGFR. One particularly attractive feature of CD19 and CD34 is that the existing Miltenyi CliniMACS screening system is available for clinical grade sorting. A human epidermal growth factor receptor (EGFR) is a tyrosine kinase receptor of the growth factor receptor ErbB family, and is not expressed on cells of hematopoietic and lymphatic systems. Cetuximab (Erbitux) is an antibody drug targeting the EGFR, and has been cleared by the U.S. FDA for the therapy of metastatic colorectal cancer and head and neck cancer. A truncated EGFR (EGFRt) can serve as a marker for the genetically modified immune cells. In EGFRt, the extracellular domains I and II and intracellular signal domain thereof are deleted, and the cetuximab binding site of the extracellular domain III thereof is retained. EGFRt can be used for immunomagnetic enrichment and flow cytometry and immunohistochemical detection by genetically modified T cells as markers, and removing in-vivo genetically modified T cells with the cetuximab. Although these above surface proteins can serve as marker genes, they all have a relatively large molecular weight, thereby possibly reducing the packaging capacity and transduction efficiency of a gene vector, and affecting the expression density of a CAR on T cells, the target antigen binding ability and the tumor killing effect.

Compared with these above large molecular marker genes, the use of a human B cell maturation antigen extracellular domain (tBCMA) polypeptide for marking genetically modified T cells has the following advantages:

1) Compared with a large molecular marker gene, an extracellular domain using BCMA only has 54 aa, which is several times smaller than CD19 and EGFR used as a marker gene, thus, the gene load can be effectively reduced when a gene vector is constructed, so that the gene vector has a greater advantage in the packaging capacity, transduction efficiency and the killing capacity of CAR-T cells.

2) The tBCMA is derived from the extracellular domain of BCMA. Since BCMA is a protein expressed from the human body itself, it has no immunogenicity for the human. Thus, upon infusion of genetically engineered cells carrying the tBCMA as a marker, an immune response to a marker protein will not be caused.

3) BCMA is expressed mainly when B cells are further differentiated into plasmocytes, and rarely expressed on hematopoietic stem cells, unmodified T cells and immature B cells. Thus, the tBCMA, as a marker, can be used for the removing of the genetically engineered cells after infusion, without causing damage to vital tissues and organs of the human body. Currently, a variety of drugs targeting BCMA, including antibody-drug conjugate (ADC), bispecific T cell engager (BiTE) and chimeric antigen receptor modified T cell (CAR-T) therapy, are all in active clinical development. Recently, the U.S. FDA approved BCMA-targeting ADC drug, i.e., Belantamab mafodotin-blmf (GSK2857916), and BCMA-targeting CAR-T cell therapy, i.e., Abecma (idecabtagene vicleucel), for the therapy of relapsed or refractory multiple myeloma. These drugs can be used effectively for removing genetically engineered cells using the tBCMA as a marker during infusion.

Therefore, the present application uses a tBCMA polypeptide as a marker for the development of the detection and removing of universal genetically engineered cells, and has wide application prospects in genetically engineered cells, particularly in genetically engineered immune cells represented by CAR-T and CAR-NK cells.

The present disclosure uses a tBCMA polypeptide as a marker for the development of the detection and removing of genetically engineered cells. The genetically engineered cells may be any immune cell modified by genetic engineering, including but not limited to a T cell, a γδT cell, an NK cell, an NKT cell or a macrophage; and a T cell, a γδT cell, an NK cell, an NKT cell or a macrophage from induced pluripotent stem cells.

### Embodiment 1. CD70 has higher expression on a variety of tumor cells and can serve as a target for CAR-T cell therapy

This embodiment used a CD70 antibody (Anti-CD70-PE, BD Pharminggen, Cat#555835) to perform flow cytometry detection on tumor cell lines of various tissue sources, and a CD70 flow cytometry detection reagent was Anti-CD70-PE. A specific experimental method was as follows: firstly, 2 × 10⁵ to 1 × 10⁶ cells/tube was taken and placed in 1 ml of a flow cytometry buffer solution (PBS, 2% FBS), and the mixture was subjected to vortex for even mixing, and centrifuged at 400 ×G for 5 minutes; the supernatant was removed, 50 µl of a flow cytometry buffer solution was added, 1 to 2 µl of a corresponding detection antibody reagent was added, and the mixture was subjected to vortex for even mixing, and placed on ice for incubation for 30 minutes; 1 ml of a flow cytometry buffer solution was added, the mixture was subjected to vortex for even mixing, and centrifuged at 400 × G for 5 minutes; and the supernatant was removed, 300 to 500 µl of a flow cytometry buffer solution was added, and the mixture was subjected to vortex for even mixing, and subjected to detection and analysis with a flow cytometry analyzer. Results show that CD70 is positively expressed in a variety of tumor cell lines, including myeloid leukemia cell lines THP-1, MOLM-13 and U937, and hepatoma cell lines NCI-H460 and 95D (FIG. 1A), but negatively expressed in T-cell leukemia cell lines CCRF-CEM and MOLT-4, and hepatoma cell lines NCI-H727, NCI-H1299 and PC-9 (FIG. 1B). The cell lines used in this embodiment can all be purchased publicly.

### Embodiment 2. Since CD27 has a higher expression on T cells, the use of this antibody cannot effectively perform flow cytometry detection on CAR-T cells using CD27 as an antigen recognition domain

This embodiment first constructed two CAR lentiviral vectors, i.e., CD27FL-CD3 ζ-002 and CD27FL BB-002, using full-sequence CD27 (CD27FL) as an antigen binding domain. A vector structure of CD27FL-CD3 ζ-002 is shown in A in FIG. 7, where the amino acid sequences of the CD27FL and a CD3 ζ T cell stimulation domain are as shown in SEQ ID NO. 6 and SEQ ID NO. 27, respectively. For another vector, a vector structure of CD27FL BB-002 is shown in E in FIG. 7, where the amino acid sequences of the CD27FL, a 4-1BB co-stimulation domain and a CD3 ζ T cell stimulation domain are as shown in SEQ ID NO. 6, SEQ ID NO. 26 and SEQ ID NO. 27, respectively.

CAR-T cells were obtained by CAR lentiviral vector transduction, and subjected to flow cytometry detection with a CD27 antibody (Anti-CD27-APC, BD Pharminggen, Cat#555440) (FIG. 2A-FIG. 2B). A specific method for lentiviral vector production was as follows: firstly, at Day 1, HEK 293T cells were seeded at 5 × 10E6/10 cm dish; at Day 2, psPAX2, pMD2G, and CAR sequence-bearing transport plasmids CD27FL-CD3 ζ-002 or CD27FL BB-002 were mixed evenly with 36 µl of 1 mg/ml polyethylenimine (branched polyethylenimine) at 12 µg/10 cm dish at a ratio of 2:1:4, respectively, and then subjected to three-plasmid transfection; at Day 3, medium change was conducted with a DMEM complete medium (DMEM, 10% FBS, 10 unit/ml gentamycin) at 10 ml/10 cm dish; at Day 4 to Day 5, a culture solution was collected twice, respectively, and subjected to low-speed centrifugation at 500 ×G at 4°C for 10 minutes to remove cell debris and impurities; and finally, the viral vector was concentrated by ultracentrifugation at 25000 ×G for 2 hours, and subpackaged and stored in a refrigerator at -80°C. A specific method for lentiviral vector transduction was as follows: firstly, collected and activated T cells were used, where the density of the T cells was 2 × 10⁶ cells/ml, and the concentration of IL2 was 300 U/ml, CD3/CD28 antibody coupled magnetic beads (ThermoFisher, Beijing T&L Biological Technology Co., Ltd.) were added in a ratio of 2:1 to cells, and cell culture was conducted under conditions of 37°C and 5% CO₂; 24 hours later, the concentrated lentiviral vector was used for T cell transduction, where the density of the T cells was 5 × 10⁶ cells/ml, the concentration of polybrene was µg/ml, and the concentration of IL2 was 300 U/ml; 4 hours later, solution supplementation was conducted until the cell density was 2 × 10⁶ cells/ml; and 72 hours later, centrifugation was conducted at 400 ×G for 5 minutes, a supernatant was removed, a T cell culture solution (RPMI 1640, 10% FBS, IL2 300 U/ml) was added, and cell culture amplification was conducted at a cell density of 1 × 10⁶ cells/ml. After culture for 2 to 4 days, flow cytometry analysis was conducted, to detect the transduction efficiency. The specific method for flow cytometry detection was the same as that in Embodiment 1. Results show that CD27 can also be detected to be highly expressed on untransduced T cells, indicating that CD27 is highly expressed on T cells, and therefore CAR-T cells using CD27 as an antigen binding domain are not suitable for flow cytometry detection using a CD27 antibody.

### Embodiment 3. CAR-T cells using CD27 as an antigen recognition domain can be effectively detected by using a tBCMA polypeptide as a marker, and this marker does not affect a tumor killing function thereof.

This embodiment first used a lentiviral vector carrying and expressing CD70 to transduce a HeLa cell line, and a HeLa-CD70 cell line expressing CD70 was obtained by flow cytometry sorting (FIG. 3A). An amino acid sequence of the CD70 is as shown in SEQ ID NO. 5. The specific method for lentiviral vector production was the same as that in Embodiment 2, where a transport plasmid used carried a sequence expressing CD70. Three CAR-T cells targeting CD70, i.e., tBCMA-CD27FL-CD3z-002, CD27FL-tBCMA-CD3z-002 and CD27FL-CD3z-tBCMA-002, using tBCMA polypeptide as a marker were obtained by CAR lentiviral vector transduction (FIG. 3B, B to D in FIG. 7). The specific method for lentiviral vector production was the same as that in Embodiment 2. The specific methods for lentiviral vector transduction and flow cytometry detection were the same as those in Embodiment 2 and Embodiment 1, respectively. In-vitro killing assays were conducted with an iCELLigence^{™} real-time cell analyzer (Agilent Biosciences, Inc.) (FIG. 3C). A specific method for the in-vitro killing assays was as follows: firstly, tumor target cells were added into a 96-well plate provided by the real-time cell analyzer, with 1 × 10⁵ cells per well at 2 × 10⁵ cell/mL; 24 hours later, T or CAR-T cells were added at an effect-target ratio (E:T ratio) of 1:1; and data were collected after 48 hours of co-culture. An amino acid sequence of the tBCMA polypeptide is as shown in SEQ ID NO. 4. Results show that the tBCMA polypeptide can be linked, via a linker peptide, to the antigen recognition domain of the CAR to form a fusion protein, or can be linked via T2A, and expressed simultaneously with the CAR on the CAR-T cell membrane, and can be effectively used as a marker for the flow cytometry detection of the CAR-T cells. Meanwhile, the CAR-T cells using the tBCMA polypeptide as the marker do not affect a tumor cell killing function thereof. Among the three CAR-T cells using the tBCMA polypeptide as the marker and targeting CD70, the CAR-T cells obtained by transduction with the tBCMA-CD27FL-CD3z-002 vector perform best in flow cytometry detection and target cell killing.

### Embodiment 4. A tBCMA polypeptide may serve as a universal marker for detection marking of a variety of CAR-T cells.

This embodiment first established HeLa cell lines expressing BCMA, BAFF-R, TACI, CD20 and CD30, i.e., HeLa-BCMA, HeLa-BAFF-R, HeLa-TACI, HeLa-CD20, HeLa-CD30, and NCI-H1299 expressing CD276 (FIG. 4A). Amino acid sequences of BCMA, BAFF-R, TACI, CD20, CD30 and CD276 are shown as SEQ ID NO. 3, SEQ ID NO. 22, SEQ ID NO. 23, SEQ ID NO. 13, and SEQ ID NO. 7, SEQ ID NO. 9, respectively. CAR-T cells using the tBCMA polypeptide as the marker were prepared by lentiviral vector transduction of T cells, i.e., tBCMA-APRIL-BB-002, tBCMA-BAFFs2-BB-002, tBCMA-CD20 BB-002, tBCMA-CD30LR-CD3z-002 and tBCMA-CD276L-BB-002 (FIG. 4B to FIG. 4F). The specific method for lentiviral vector production was the same as that in Embodiment 2. The specific methods for lentiviral vector transduction and flow cytometry detection were the same as those in Embodiment 2 and Embodiment 1, respectively. Flow cytometry detection reagents for tumor antigens BCMA, BAFF-R, TACI, CD20, CD30 and CD276 were Anti-human BCMA-PE (Biolegend, Cat#357503), anti-human BAFF-R-APC/Cyanine7 (Biolegend, Cat#316912), eGFP (TACI-expressed reporter gene), anti-human CD20-APC (BD, Cat#559776), anti-human CD30-FITC (BD, Cat#555829), and anti-human CD276-APC (Biolegend, Cat#351006), respectively; flow cytometry detection reagents for APRIL, BAFF, CD20 scFV, CD30L and tBCMA polypeptide-marked CAR-T cells are anti-human APRIL-Biotin (Biolegend, Cat#524903)/ Streptavidin-APC (BD, Cat#554067), anti-human BAFF-PE (Biolegend, Cat#318605), Biotin-SP-conjugated AffiniPure F (ab') Fragment Goat Anti-Human IgG (H+L) (Jackson ImmunoResearch, Cat# 109-066-006/ Streptavidin-APC (BD, Cat#554067)), Human CD30 Ligand/TNFSF8 APC-conjugated Antibody (R&D, Cat#FAB1028A), and Anti-Human BCMA-PE (Biolegend, Cat#357503), respectively; CD276L expression was not assessed as no efficient flow cytometry antibodies were found.

In-vitro killing assays were conducted on HeLa tumor cells carrying corresponding targets with an iCELLigence^{™} real-time cell analyzer (Agilent Biosciences, Inc.) (FIG. 4G to FIG. 4I). The specific method for the in-vitro killing assays was the same as that in Embodiment 3, where targets cells used were HeLa-BCMA, HeLa-TACI, HeLa-BAFF-R, HeLa-CD20, HeLa-CD30, and NCI-H1299, respectively. The APRIL, BAFFs2, CD20 scFv, CD30LR and CD276L were antigen binding domains of tBCMA-APRIL-BB-002, tBCMA-BAFFs2-BB-002, tBCMA-CD20 BB-002, tBCMA-CD30LR-CD3z-002 and tBCMA-CD276L-BB-002 CAR-T cells, respectively. Amino acid sequences thereof are as shown in SEQ ID NO. 24, SEQ ID NO. 25, SEQ ID NO. 14, SEQ ID NO. 8, and SEQ ID NO. 10, respectively.

Results show that the tBCMA polypeptide as the marker can be used extensively for the flow cytometry detection of a variety of CAR-T cells. Meanwhile, the CAR-T cells using the tBCMA polypeptide as the marker do not affect a tumor cell killing function thereof. However, this marker cannot be used in a case where antigen binding domains of the CAR-T cells are BCMA natural ligands APRIL and BAFF. In this embodiment, tBCMA-APRIL-BB-002 and tBCMA-BAFFs2-BB-002 CAR-T cells using the tBCMA polypeptide as the marker used BCMA natural ligands APRIL (a proliferation inducing ligand) and BAFF (B-cell activating factor) as antigen binding domains, respectively. Although the CAR-T cells can be efficiently detected by BCMA antibodies (FIG. 4B to FIG. 4C), since the receptors and ligands constituting the two CAR molecules bind to each other, tBCMA-APRIL-BB-002 CAR-T cells cannot effectively kill HeLa tumor cells carrying BCMA and TACI (FIG. 4G), or tBCMA-BAFFs2-BB-002 CAR-T cells cannot effectively kill HeLa tumor cells carrying BCMA, BAFF-R and TACI (FIG. 4H). However, since the currently discovered BCMA natural ligands are only APRIL and BAFF, the limitation of using the tBCMA polypeptide as the marker for the CAR-T cells is very small. Therefore, it can be predicted that as a detection marker and a removing target, the tBCMA polypeptide has broad prospects in genetically modified immune cell therapy.

### Embodiment 5. A tBCMA polypeptide can be used for removing CAR-modified tumor cells targeting CD70

This embodiment first established a CAR tumor cell line expressing a tBCMA polypeptide marker, i.e., HeLa-tBCMA-CD27FL-CD3z-002 (FIG. 5A), and CAR-T cells expressing and targeting BCMA, i.e., YK-BCMA BB-002, were obtained by lentiviral vector transduction (FIG. 5B). For the vector construction and cell preparation for the YK-BCMA BB-002 CAR-T cells, refer to the following document: Risk Factors Associated with Durable Progression-Free Survival in Patients with Relapsed or Refractory Multiple Myeloma Treated with Anti-BCMA CAR T-cell Therapy. Clin Cancer Res. 2021 Dec 1;27(23):6384-6392. The specific method for lentiviral vector production was the same as that in Embodiment 2. The specific methods for lentiviral vector transduction and flow cytometry detection were the same as those in Embodiment 2 and Embodiment 1, respectively. The CAR-T cells targeting BCMA can effectively kill in vitro the CAR tumor cell line HeLa-tBCMA-CD27FL-CD3z-002 using the tBCMA polypeptide as the marker (FIG. 5C). For the specific method for the in-vitro killing assays, refer to Embodiment 3. Results show that the tBCMA polypeptide can be used as a marker for the flow cytometry detection of CD70 CAR-T cells. Meanwhile, the tBCMA polypeptide can also be used as a target for removing of tBCMA polypeptide marker cells. This result further suggests that the tBCMA polypeptide as the marker can be used not only for the detection of CAR-T cells, but also for the removing of CAR-T cells.

### Embodiment 6. A tBCMA polypeptide may serve as a universal marker for removing of CAR expression cells

This embodiment first established CAR tumor cell lines expressing a tBCMA polypeptide marker, i.e., HeLa-tBCMA-CD20-BB-002, HeLa-tBCMA-CD30LR-CD3z-002 and HeLa-tBCMA-CD276L-BB-002 (FIG. 6A), and CAR-T cells expressing and targeting BCMA, i.e., YK-BCMA BB-002, were obtained by lentiviral vector transduction (FIG. 6B). The source of the YK-BCMA BB-002 CAR-T cells was the same as that in Embodiment 5. The specific method for lentiviral vector production was the same as that in Embodiment 2. The specific methods for lentiviral vector transduction and flow cytometry detection were the same as those in Embodiment 2 and Embodiment 1, respectively. The CAR-T cells targeting BCMA can effectively kill in vitro the CAR tumor cell lines HeLa-tBCMA-CD20-BB-002, HeLa-tBCMA-CD30LR-CD3z-002 and HeLa-tBCMA-CD276L-BB-002 using the tBCMA polypeptide as the marker (FIG. 6C). For the specific method for the in-vitro killing assays, refer to Embodiment 3. Results show that the tBCMA polypeptide can be used as a marker for the flow cytometry detection of CAR-T cells. Meanwhile, the tBCMA polypeptide can also be used as a target for removing of tBCMA polypeptide marker cells. This result further suggests that the tBCMA polypeptide as the marker can be used not only for the detection of CAR-T cells but also for the removing of CAR-T cells.

The present disclosure uses tBCMA as a marker for detecting and removing genetically engineered immune cells, and has the following characteristics: 1) the tBCMA is several times smaller than the existing marker genes, so that the gene load can be effectively reduced, and the transduction efficiency is improved; 2) since the tBCMA has no immunogenicity for the human, upon infusion of genetically engineered cells carrying the tBCMA as a marker, an immune response to a marker protein will not be caused; and 3) due to the limitation of BCMA expression, when the tBCMA as a marker is used for the removing of the genetically engineered cells after infusion, damage to vital tissues and organs of the human body will not be caused. Therefore, the present disclosure uses the tBCMA as the marker gene to provide a better method for detecting and removing genetically engineered immune cells, so that a vector carrying the marker gene can be further enhanced in terms of packaging capacity and transduction efficiency, the current problem of removing the genetically engineered cells after detection and infusion can be solved, and the genetically engineered immune cells have a greater advantage in terms of tumor killing capacity.

The present disclosure uses an extracellular domain polypeptide (tBCMA) of a B cell maturation antigen as a marker for the technology of detecting and removing the genetically engineered immune cells. The present disclosure can be widely used for the detection of various genetically engineered immune cells, thereby effectively solving the problems of detection of the genetically engineered immune cells after preparation and infusion, and providing a feasible strategy when these cells need to be removed due to serious toxic and side effects in clinical therapy.

The specific embodiments of the present disclosure are described above. It needs to be understood that the present disclosure is not limited to the specific implementations mentioned above. Those skilled in the art can make various changes or modifications within the scope of the claims, which does not affect the substantive content of the present invention. The embodiments in the present application and the features in the embodiments can be combined randomly with each other in the case of no conflict.

## Claims

1. A gene tBCMA for a B cell maturation antigen (BCMA) extracellular domain, wherein the gene tBCMA is a gene encoding a complete or partial sequence of BCMA extracellular domain, or a gene encoding a sequence at least 85% identical to the complete or partial sequence, and the complete nucleotide sequence of the gene tBCMA is as shown in SEQ ID NO. 2.

2. A tBCMA polypeptide for BCMA extracellular domain, wherein the tBCMA polypeptide is a polypeptide encoded by the gene tBCMA for BCMA extracellular domain according to claim 1, and the complete polypeptide amino acid sequence encoded by the gene tBCMA is as shown in SEQ ID NO. 4.

3. A recombinant expression vector, comprising a nucleotide sequence of the gene tBCMA according to claim 1.

4. A genetically engineered cell, comprising the gene tBCMA for BCMA extracellular domain according to claim 1.

5. The genetically engineered cell according to claim 4, wherein the genetically engineered cell is an immune cell modified by genetic engineering.

6. The genetically engineered cell according to claim 5, wherein the immune cell comprises a T cell, a γδT cell, an NK cell, an NKT cell, or a macrophage; and a T cell, a γδT cell, an NK cell, an NKT cell, or a macrophage from induced pluripotent stem cells.

7. The genetically engineered cell according to claim 6, wherein the T cell is a chimeric antigen receptor modified T cell (CAR-T cell), and the CAR-T cell comprises the gene tBCMA for BCMA extracellular domain according to claim 1 as a marker gene.

8. The genetically engineered cell according to claim 7, wherein the CAR-T cell is a CAR-T cell where an antigen binding domain targets any of the following targets comprising but not limited to: CD1a, CD2, CD4, CD5, CD7, CD19, CD20, CD22, CD30, CD32b, CD33, CD34, CD37, CD38, CD44v6, CD56, CD70, CD79b, CD83, CD117, CD123, CD133, CD138, CD155, CD171, CD276, CD319, CD371, Chlorotoxin, FLT3, Folate receptor beta, GPRC5D, IL1RAP, Lag-3, LewY, Sigelec-6, LILRB4, LMP1, B7H6, NKG2DLs, PD-L1, ROR1, TIM3, CEA, Claudin-6, Claudin-18.2, EGFR, EGFRvIII, EpCam, EphA2, Fibroblast activation protein alpha, GTPase-activating protein, GD2, Glypican-1, GPC2, GPC3, Her2, IL13Ra2, Mesothelin, PSCA, PSMA, Muc1, and TAG-72.

9. The genetically engineered cell according to claim 8, wherein the CAR-T cell is a CAR-T cell where CD27 as an antigen binding domain targets the CD70.

10. The genetically engineered cell according to claim 9, wherein the antigen binding domain is a complete or partial sequence of the CD27, or a sequence 85% identical to the complete or partial sequence of the antigen binding domain CD27, and the complete amino acid sequence of the CD27 is as shown in SEQ ID NO. 6.

11. The genetically engineered cell according to claim 8, wherein the CAR-T cell is a CAR-T cell where a CD30 ligand as an antigen binding domain targets the CD30.

12. The genetically engineered cell according to claim 11, wherein the antigen binding domain is a complete or partial sequence of the CD30 ligand, or a sequence 85% identical to the complete or partial sequence of the antigen binding domain CD30 ligand, and the complete amino acid sequence of the CD30 ligand is as shown in SEQ ID NO. 8.

13. The genetically engineered cell according to claim 8, wherein the CAR-T cell is a CAR-T cell where a CD276 ligand as an antigen binding domain targets the CD276.

14. The genetically engineered cell according to claim 13, wherein the antigen binding domain is a complete or partial sequence of the CD276 ligand, or a sequence 85% identical to the complete or partial sequence of the antigen binding domain CD276 ligand, and the complete amino acid sequence of the CD276 ligand is as shown in SEQ ID NO. 10.

15. The genetically engineered cell according to claim 8, wherein the CAR-T cell is a CAR-T cell where IL3 as an antigen binding domain targets the CD123.

16. The genetically engineered cell according to claim 15, wherein the antigen binding domain is a complete or partial sequence of the IL3, or a sequence 85% identical to the complete or partial sequence of the antigen binding domain IL3, and the complete amino acid sequence of the IL3 is as shown in SEQ ID NO. 12.

17. The genetically engineered cell according to claim 8, wherein the CAR-T cell is a CAR-T cell where a CD20 single-chain antibody as an antigen binding domain targets the CD20.

18. The genetically engineered cell according to claim 17, wherein the antigen binding domain is a complete or partial sequence of the CD20 single-chain antibody, or a sequence 85% identical to the complete or partial sequence of the antigen binding domain CD20 single-chain antibody, and the complete amino acid sequence of the CD20 single-chain antibody is as shown in SEQ ID NO. 14.

19. The genetically engineered cell according to claim 7, wherein in the CAR-T cell, a tBCMA polypeptide expressed from the gene tBCMA for BCMA extracellular domain is capable of being directly linked to an antigen binding domain of a chimeric antigen receptor (CAR) to form a fusion protein.

20. The genetically engineered cell according to claim 7, wherein in the CAR-T cell, a tBCMA polypeptide expressed from the gene tBCMA for BCMA extracellular domain is linked to an antigen recognition region of a CAR via a linker peptide to form a fusion protein.

21. The genetically engineered cell according to claim 20, wherein the amino acid sequence of the linker peptide comprises but is not limited to SEQ ID NO. 15, SEQ ID NO. 16, SEQ ID NO. 17, or SEQ ID NO. 18.

22. The genetically engineered cell according to claim 7, wherein in the CAR-T cell, a tBCMA polypeptide expressed from the gene tBCMA for BCMA extracellular domain is linked via a 2A peptide, and expressed simultaneously with a CAR on a CAR-T cell membrane.

23. The genetically engineered cell according to claim 22, wherein the amino acid sequence of the 2A peptide comprises but is not limited to SEQ ID NO. 19, SEQ ID NO. 20, or SEQ ID NO. 21.

24. The genetically engineered cell according to claim 7, wherein in the CAR-T cell, the gene tBCMA for BCMA extracellular domain is linked via an internal ribosome entry site (IRES), and expressed simultaneously with a CAR on a CAR-T cell membrane.

25. The genetically engineered cell according to claim 7, wherein in the CAR-T cell, a tBCMA polypeptide expressed from the gene tBCMA for BCMA extracellular domain is expressed, via a gene editing method, simultaneously with a CAR on a CAR-T cell membrane.

26. The genetically engineered cell according to claim 25, wherein a gene editing tool used by the gene editing method is transcription activator-like effector nucleases (TALENs) or clustered regularly interspaced short palindromic repeats/CRISPR-associated protein 9 (CRISPR/Cas9).

27. The genetically engineered cell according to claim 7, wherein in the CAR-T cell, a tBCMA polypeptide expressed from the gene tBCMA for BCMA extracellular domain is expressed, via multi-vector co-transduction or sequential transduction, simultaneously with a CAR on a CAR-T cell membrane.

28. The genetically engineered cell according to claim 6, wherein the NK cell is a chimeric antigen receptor modified NK cell (CAR-NK cell), and the CAR-NK cell comprises the gene tBCMA for BCMA extracellular domain according to claim 1 as a marker gene.

29. A use of the gene tBCMA for BCMA extracellular domain according to claim 1 in detecting a genetically engineered cell, wherein the genetically engineered cell comprises the gene tBCMA for BCMA extracellular domain according to claim 1.

30. The use of the gene tBCMA for BCMA extracellular domain in detecting the genetically engineered cell according to claim 29, wherein a detection of the genetically engineered cell is completed by a detection reagent capable of specifically recognizing a tBCMA marker gene.

31. The use of the gene tBCMA for BCMA extracellular domain in detecting the genetically engineered cell according to claim 30, wherein the detection reagent comprises a small molecular compound, a polypeptide, a protein, or an antibody.

32. The use of the gene tBCMA for BCMA extracellular domain in detecting the genetically engineered cell according to claim 29, wherein the genetically engineered cell is an immune cell modified by genetic engineering.

33. The use of the gene tBCMA for BCMA extracellular domain in detecting the genetically engineered cell according to claim 32, wherein the immune cell comprises a T cell, a γδT cell, an NK cell, an NKT cell, or a macrophage; and a T cell, a γδT cell, an NK cell, an NKT cell, or a macrophage from induced pluripotent stem cells.

34. The use of the gene tBCMA for BCMA extracellular domain in detecting the genetically engineered cell according to claim 33, wherein the T cell is a CAR-T cell, and the CAR-T cell comprises the gene tBCMA for BCMA extracellular domain according to claim 1 as a marker gene.

35. The use of the gene tBCMA for BCMA extracellular domain in detecting the genetically engineered cell according to claim 33, wherein the NK cell is a CAR-NK cell, and the CAR-NK cell comprises the gene tBCMA for BCMA extracellular domain according to claim 1 as a marker gene.

36. A use of the gene tBCMA for BCMA extracellular domain according to claim 1 in removing a genetically engineered cell, wherein the genetically engineered cell comprises the gene tBCMA for BCMA extracellular domain according to claim 1.

37. The use of the gene tBCMA for BCMA extracellular domain in removing the genetically engineered cell according to claim 36, wherein removing of the genetically engineered cell is completed by a removing reagent specifically recognizing a tBCMA marker gene.

38. The use of the gene tBCMA for BCMA extracellular domain in removing the genetically engineered cell according to claim 37, wherein the removing reagent comprises a small molecular compound, a polypeptide, a protein, an antibody, or a genetically engineered immune cell targeting tBCMA.

39. The use of the gene tBCMA for BCMA extracellular domain in removing the genetically engineered cell according to claim 36, wherein the genetically engineered cell is an immune cell modified by genetic engineering.

40. The use of the gene tBCMA for BCMA extracellular domain in removing the genetically engineered cell according to claim 39, wherein the immune cell comprises a T cell, a γδT cell, an NK cell, an NKT cell, or a macrophage; and a T cell, a γδT cell, an NK cell, an NKT cell, or a macrophage from induced pluripotent stem cells.

41. The use of the gene tBCMA for BCMA extracellular domain in removing the genetically engineered cell according to claim 40, wherein the T cell is a CAR-T cell, and the CAR-T cell comprises the gene tBCMA for BCMA extracellular domain according to claim 1 as a marker gene.

42. The use of the gene tBCMA for BCMA extracellular domain in removing the genetically engineered cell according to claim 40, wherein the NK cell is a CAR-NK cell, and the CAR-NK cell comprises the gene tBCMA for BCMA extracellular domain according to claim 1 as a marker gene.
